# EUROPEAN PATENT APPLICATION

(11) **EP 2 907 511 A1**
(43) Date of publication of application: **19.08.2015**
(21) Application number: 15158251.7
(22) Date of filing: 21.12.2009
(51) Int. Cl.: A61K 31/137, A61P 37/00, A61P 37/06

(54) **DOSAGE REGIMEN FOR A S1P RECEPTOR AGONIST**

(30) Priority: 22.12.2008 US 139672 P; 19.06.2009 US 218530 P; 29.09.2009 US 246715 P
(62) Divisional of application: 09797234.3
(71) Applicant: Novartis AG, 4056 Basel (CH)
(72) Inventor: Schmouder, Robert, East Hanover, NJ New Jersey 07936 (US); Dumortier, Thomas, CH-4002 BASEL (CH); David, Olivier, CH-4002 BASEL (CH); Looby, Michael, CH-4002 BASEL (CH)
(74) Representative: Rouquayrol, Céline Hélène

(57) **Abstract**

S1P receptor modulators or agonists are administered following a dosage regimen whereby during the initial days of treatment the daily dosage is lower than the standard daily dosage.

## Description

The present invention relates to a dosage regimen of a S1P receptor modulator or agonist. More specifically, the present invention relates to a dosage regimen for the treatment of patients suffering from autoimmune diseases or disorders, such as, for example, multiple sclerosis with a S1P receptor modulator or agonist.

S1P receptor modulators or agonists are compounds which signal as agonists at one or more sphingosine-1 phosphate receptors, for example, S1P1 to S1P8. The binding of an agonist to a S1P receptor may, for example, result in the dissociation of intracellular heterotrimeric G-proteins into Gα-GTP and Gβγ-GTP, and/or the increased phosphorylation of the agonist-occupied receptor, and/or the activation of downstream signaling pathways/kinases.

S1P receptor modulators or agonists are useful therapeutic compounds for the treatment of various conditions in mammals, especially in human beings. For example, the efficacy of S1P receptor modulators or agonists in the prevention of transplant rejection has been demonstrated in rat (skin, heart, liver, small bowel), dog (kidney), and monkey (kidney) models. In addition, due to their immune-modulating potency, S1P receptor modulators or agonists are also useful for the treatment of inflammatory and autoimmune diseases. In particular, the efficacy of the S1P receptor agonist FTY720 in the treatment of multiple sclerosis has been demonstrated in humans (as described in, for example, "FTY720 therapy exerts differential effects on T cell subsets in multiple sclerosis". Mehling M, Brinkmann V, Antel J, Bar-Or A, Goebels N, Vedrine C, Kristofic C, Kuhle J, Lindberg RL, Kappos L. Neurology. 2008 Oct 14;71(16):1261-7; and "Oral fingolimod (FTY720) for relapsing multiple sclerosis". Kappos L, Antel J, Comi G, Montalban X, O'Connor P, Polman CH, Haas T, Korn AA, Karlsson G, Radue EW; FTY720 D2201 Study Group. N Engl J Med. 2006 Sep 14;355(11):1124-40.).

Multiple sclerosis is the chief cause of neurological disability in young adults and the most common demyelinating disorder of the central nervous system. Currently available therapies, such as interferon-β and glatiramer acetate, only have modest efficacy and therefore demonstrate only marginal effects on the progression of the disease. Furthermore, these biological agents are administered parenterally and are associated with some adverse effects such as, for example, localized reactions at the injection site and pyretic symptoms. Therefore, there is a strong medical need for an effective oral treatment for multiple sclerosis. S1 P receptor modulators or agonists may produce a negative chronotropic effect, i.e. they may reduce the cardiac rhythm, as described e.g. in "FTY720: Placebo-Controlled Study of the Effect on Cardiac Rate and Rhythm in Healthy Subjects", Robert Schmouder, Denise Serra, Yibin Wang, John M. Kovarik, John DiMarco, Thomas L. Hunt and Marie-Claude Bastien. J. Clin. Pharmacol. 2006; 46; 895. Administration of 1.25 mg of FTY720 may induce a decrease in heart rate of approximately 8 beats/min (BPM).

As a consequence of this side effect, the S1 P modulator or agonist therapy may have to be initiated under close medical supervision in order to check that the cardiac rhythm is maintained at an acceptable level. This may involve the hospitalisation of patients, which makes the treatment more expensive and complicated.

Therefore, there is a need to reduce the negative chronotropic side effect that may be generated by the administration of S1 P receptor modulators or agonists, while maintaining the ability to administer an adequate dosage in order to treat or prevent the diseases for which the compound is administered. There is furthermore a need to enhance patient compliance.

### Brief Disclosure of the Invention

Surprisingly it has been found that by administering the S1 P receptor modulator or agonist according to a specific dosage regimen, it is possible to reduce side effects which may be associated with the administration of such compounds. For example, administering a S1 P receptor agonist or modulator according to the specific dosage regimen of the present invention may significantly, reduce or even completely eliminate, the negative chronotropic side effect. In particular it may avoid an abrupt drop in the heart rate.

Administering a S1 P receptor agonist or modulator according to the specific dosage regimen of the present invention may also significantly reduce or even completely eliminate the risks that the patients taken the S1 P receptor agonist or modulator suffer from atrio-ventricular (AV) blocks or heart pause.

Furthermore the specific dosage regimen of the present invention permits to administer a S1 P receptor agonist or modulator to categories of patients for which the ratio risk/benefit may otherwise be less favourable. Such patients are for example patients susceptible to or suffering from heart failure or arrythmias, patients with high grade atrio-ventricular blocks or sick sinus syndrome, patients with a history of syncopal episodes, or patients under beta blockers or anti-arrhythmic treatment, such as patients under anti-arrhythmic drugs; or patients that have undergone an interruption or treatment holiday in the maintenance dosage regime e.g. a holiday of greater than 4 days, greater than 6, 8, 10, 12 or 14 days.

The dosage regimen of the present invention is a regimen for the initiation of S1P receptor modulator or agonist therapy, which enables the standard daily therapeutic dosage range of the S1 P receptor to be achieved with minimal negative chronotropic effects and/or the AV block effects possibly associated with S1 P receptor modulator therapy.

### S1P receptor modulators or agonists

Preferred S1 P receptor agonists or modulators are, for example, compounds which, in addition to their S1 P binding properties, also have accelerating lymphocyte homing properties. For example, the compounds may elicit lymphopenia resulting from a redistribution of lymphocytes from the circulation to the secondary lymphatic tissue, which is preferably reversible, without evoking a generalized immunosuppression. Suitably, naïve cells are sequestered and CD4 and CD8 T-cells and B-cells from the blood are stimulated to migrate into lymph nodes (LN) and Peyer's patches (PP).

S1 P receptor modulators or agonists are typically sphingosine analogues, such as 2-substituted 2-amino- propane-1,3-diol or 2-amino-propanol derivatives.

In an embodiment of the invention, the S1 P receptor modulator or agonist is a compound comprising a group of formula X 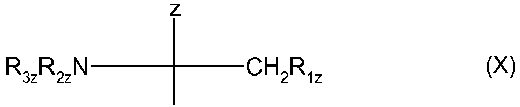 wherein Z is H, C₁₋₆alkyl, C₂₋₆alkenyl, C₂₋₆alkynyl, phenyl, phenyl substituted by OH, C₁₋₆alkyl substituted by 1 to 3 substituents selected from the group consisting of halogen, C₃₋₈cycloalkyl, phenyl and phenyl substituted by OH, or CH₂-R_{4z} wherein R_{4z} is OH, acyloxy or a residue of formula (a) 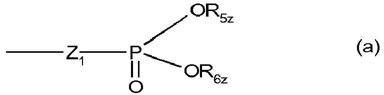 wherein Z₁ is a direct bond or O, preferably O;
each of R_{5z} and R_{6z}, independently, is H, or C₁₋₄alkyl optionally substituted by 1, 2 or 3 halogen atoms;
R_{1z} is OH, acyloxy or a residue of formula (a); and each of R_{2z} and R_{3z} independently, is H, C₁₋₄alkyl or acyl.

The group of formula X is a functional group which is attached as a terminal group to a moiety which may be hydrophilic or lipophilic and comprise one or more aliphatic, alicyclic, aromatic and/or heterocyclic residues. The resultant molecule functions as a modulator/agonist at one of more sphingosine-1-phosphate receptors.

Suitably, at least one of Z and R_{1z} is or comprises a residue of formula (a).

Examples of appropriate S1P receptor agonists or modulators include:
(i) Compounds as disclosed in EP627406A1, e.g. a compound of formula I 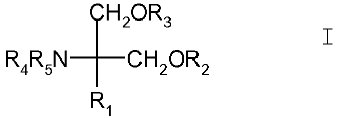 wherein R₁ is a straight- or branched (C₁₂₋₂₂)chain
   - which may have in the chain a bond or a hetero atom selected from a double bond, a triple bond, O, S, NR₆, wherein R₆ is H, C₁₋₄alkyl, aryl-C₁₋₄alkyl, acyl or (C₁₋₄alkoxy)carbonyl, and carbonyl, and/or
   - which may have as a substituent C₁₋₄alkoxy, C₂₋₄alkenyloxy, C₂₋₄alkynyloxy, arylC₁₋₄alkyl-oxy, acyl, C₁₋₄alkylamino, C₁₋₄alkylthio, acylamino, (C₁₋₄alkoxy)carbonyl, (C₁₋₄alkoxy)-carbonylamino, acyloxy, (C₁₋₄alkyl)carbamoyl, nitro, halogen, amino, hydroxyimino, hydroxy or carboxy; or
   R₁ is
   - a phenylalkyl wherein alkyl is a straight- or branched (C₆₋₂₀)carbon chain; or
   - a phenylalkyl wherein alkyl is a straight- or branched (C₁₋₃₀)carbon chain wherein said phenylalkyl is substituted by
      - a straight- or branched (C₆₋₂₀)carbon chain optionally substituted by halogen,
      - a straight- or branched (C₆₋₂₀)alkoxy chain optionally substitued by halogen,
      - a straight- or branched (C₆₋₂₀)alkenyloxy,
      - phenyl-C₁₋₁₄alkoxy, halophenyl-C₁₋₄alkoxy, phenyl-C₁₋₁₄alkoxy-C₁₋₁₄alkyl, phenoxy-C₁₋₄alkoxy or phenoxy-C₁₋₄alkyl,
      - cycloalkylalkyl substituted by C₆₋₂₀alkyl,
      - heteroarylalkyl substituted by C₆₋₂₀alkyl,
      - heterocyclic C₆₋₂₀alkyl or
      - heterocyclic alkyl substituted by C₂₋₂₀alkyl,
   and wherein the alkyl moiety may have:
   - in the carbon chain, a bond or a heteroatom selected from a double bond, a triple bond, O, S, sulfinyl, sulfonyl, or NR₆, wherein R₆ is as defined above, and
   - as a substituent C₁₋₄alkoxy, C₂₋₄alkenyloxy, C₂₋₄alkynyloxy, arylC₁₋₄alkyloxy, acyl, C_{1- 4}alkyl-amino, C₁₋₄alkylthio, acylamino, (C₁₋₄alkoxy)carbonyl, (C₁₋₄alkoxy)carbonylamino, acyloxy, (C₁₋₄alkyl)carbamoyl, nitro, halogen, amino, hydroxy or carboxy; and
   each of R₂, R₃, R₄ and R₅, independently, is H, C₁₋₄ alkyl or acyl or a pharmaceutically acceptable salt or hydrate thereof.
(ii)Compounds as disclosed in WO02/18395, e.g. a compound of formula IIa or IIb 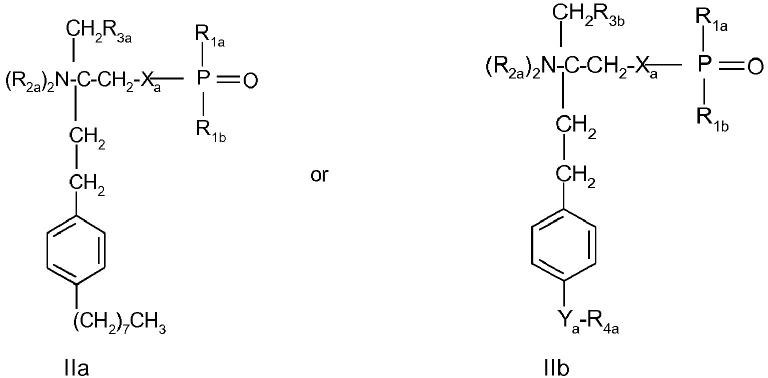 wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄₋₁₄)alkyl or (C₄₋₁₄)alkenyl;
   or a pharmaceutically acceptable salt or hydrate thereof.

When the compounds of formulae I or Ila or IIb have one or more asymmetric centers in the molecule, the present invention is to be understood as embracing the various optical isomers, as well as racemates, diastereoisomers and mixtures thereof are embraced. Compounds of formula Ila or IIb, when the carbon atom bearing the amino group is asymmetric, have preferably the R-configuration at this carbon atom.

The compounds of formulae I, IIa or IIb may exist in free or salt form. Examples of pharmaceutically acceptable salts of the compounds of the formulae I, IIa or IIb include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts of the combination of the present invention encompass hydrate and solvate forms.

In the above definitions:
- acyl may be a residue R_{y}-CO- wherein Ry is C₁₋₆alkyl, C₃₋₆cycloalkyl, phenyl or phenyl-C₁₋₄alkyl
- unless otherwise stated, alkyl, alkoxy, alkenyl or alkynyl may be straight or branched;
- aryl may be phenyl or naphthyl, preferably phenyl;
- "heterocyclic group" represents a 5- to 7 membered heterocyclic group having 1 to 3 heteroatoms selected from S, O and N. Examples of such heterocyclic groups include the heteroaryl groups indicated above, and heterocyclic compounds corresponding to partially or completely hydrogenated heteroaryl groups, e.g. furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl, pyrazinyl, tetrahydropyranyl, morpholinyl, thiomorpholinyl, pyrrolidinyl, pyrrolyl, imidazolidinyl, pyrazolidinyl, piperidinyl, piperazinyl, oxazolidinyl, isoxazolidinyl, thiazolidinyl or pyrazolidinyl. Preferred heterocyclic groups are 5-or 6-membered heteroaryl groups and the most preferred heteocyclic group is a morpholinyl, thiomorpholinyl or piperidinyl group.

When the carbon chain as R₁ is substituted in the compounds of formula I, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

A preferred compound of formula I is 2-amino-2-tetradecyl-1,3-propanediol.

A particularly preferred S1 P receptor agonist of formula I is FTY720, i.e. 2-amino-2-[2-(4-octylphenyl) ethyl]propane-1,3-diol in free form or in a pharmaceutically acceptable salt form (referred to hereinafter as Compound A), or a prodrug thereof.

In an embodiment of the invention, the agonist of formula I is FTY720 hydrochloride, as shown below:

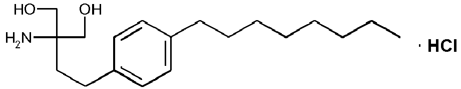

A preferred compound of formula Ila is the FTY720-phosphate (Compound B) (R₂ₐ is H, R₃ₐ is OH, Xₐ is O, R₁ₐ and R_{1b} are OH).

A preferred compound of formula IIb is the Compound C-phosphate (R₂ₐ is H, R_{3b} is OH, Xₐ is O, R₁ₐ and R_{1b} are OH, Yₐ is O and R₄ₐ is heptyl).

In a further embodiment of the invention, a S1 P receptor agonist or modulator for use in the dosage regimen of the invention may also be selective for the S1 P₁ receptor. For example, a compound which possesses selectivity for the S1 P₁ receptor over the S1P₃ receptor of at least 20 fold, e.g. 100, 500, 1000 or 2000 fold, as measured by the ratio of EC₅₀ for the S1P₁ receptor to the EC₅₀ for the S1P₃ receptor as measured by a ³⁵S-GTPγS binding assay, and wherein said compound has an EC₅₀ for binding to the S1 P1 receptor of 100 nM or less as evaluated by the ³⁵S-GTPγS binding assay.

The ³⁵S-GTPγS binding assay is described in WO03/097028 and follows the following protocol:
GTPγS binding experiments are performed as described by DS. Im et al., Mol. Pharmacol. 2000; 57:753. Ligand-mediated GTP□S binding to G-proteins is measured in GTP binding buffer (in mM: 50 HEPES, 100 NaCl, 10 MgCl₂, pH 7.5) using 25 µg of a membrane preparation from transiently transfected HEK293 cells. Ligand is added to membranes in the presence of 10 µM GDP and 0.1 nM [³⁵S]GTPγS (1200 Ci/mmol) and incubated at 30°C for 30 min. Bound GTPγS is separated from unbound using the Brandel harvester (Gaithersburg, MD) and counted with a liquid scintillation counter.

### Dosage Regimen

As previously stated, the present invention provides a novel dosage regimen which is adapted to minimize the negative chronotropic effects and/or the AV block effects possibly associated with S1 P receptor modulator or agonist therapy.

Heart effects include AV blocks, which include first degree AV blocks (e.g. PR intervals greater then 0.2 seconds) and second degree AV blocks e.g. first degree AV blocks. Heart effects include heart pauses e.g. heart pauses greater than 2 seconds.

According to the invention, there is provided the use of a S1 P receptor modulator or agonist in the manufacture of a medication, whereby said medication is administered in such a way that during the initial period of treatment the dosage is lower than the standard daily dosage and the dosage is increased, optionally stepwise, or only once, until the standard daily dosage dose is reached. Thereafter the treatment is preferably continued with the standard daily dosage of said S1 P receptor modulator or agonist.

Preferably during the initial period of treatment, the medication is administered in a dosage regimen such that daily decrease in heart rate (e.g. average or minimum daily heart rate) is acceptable or clinically not significant, or that the sinus rhythm of the patient is normal. For example, the daily decrease in heart rate (e.g. average or minimum daily heart rate) may be less than about 4bpm, e.g. less than about 3 bpm or less than about 2bpm.

The term "normal sinus rhythm" refers to the sinus rhythm of the patient when not undergoing treatment. The evaluation of normal sinus rhythm is within the ability of a physician. A normal sinus rhythm will generally give rise to a heart rate in the range from 60-100 bpm.

Preferably, during the initial period of treatment the dosage is lower than the standard daily dosage and the dosage is increased, optionally stepwise, or only once, until the standard daily dosage dose is reached. Thereafter the treatment is preferably continued with the standard daily dosage of said S1 P receptor modulator or agonist.

According to the invention, the "initial period of treatment" refers to the period during which the S1 P receptor modulator or agonist is administered at a dosage lower than the standard daily dosage. Preferably the "initial period of treatment" starts with the first administration of the S1 P receptor modulator or agonist.

The duration of the initial period of treatment may vary. In an embodiment, the initial period of treatment is up to 10 days, e.g. about a week. In a further embodiment, the initial period of treatment is 5 to 14 days, e.g. 4 to 12 days. In a further embodiment, the initial period of treatment is 7 to 10 days. In yet a further embodiment, the initial period of treatment is 4 to 7 days. In further embodiments, the initial period of treatment is selected from 10 days, or 9 days, or 8 days, or 7 days, or 6 days, or 5 days, or 4 days.

As herein above defined, standard daily dosage (also called standard daily dose) refers to the required daily maintenance dose of the drug which is given to the patients for treating or preventing the disease to be treated or prevented. Suitably, the standard daily dosage corresponds to the therapeutically effective dosage.

The therapeutically effective dosage (also called therapeutic dose) refers to the dosage of the S1 P receptor modulator or agonist which is necessary to effectively treat the intended disease or condition (i.e. so that the subject shows reduced signs or symptoms of rebound of the disease to be treated or prevented, and preferably no signs and symptoms at all).

During the initial period of treatment the S1 P receptor modulator or agonist may be administered at a dosage e.g. up to 10-fold less, e.g. up to 8-fold less, e.g. up to 4-fold less, than the standard daily maintenance dose, e.g. than the therapeutic dose.

In a preferred embodiment, there is provided the use of an S1 P receptor modulator or agonist in the manufacture of a medication, whereby said medication is administered in such a way that during the initial period of treatment (e.g. the first 10 days of treatment, e.g. the first week of treatment, or the first 5 to 14 days of treatment, the first 4 to 12 days of treatment, or the first 7 to 10 days of treatment, or the first 10, 9, 8, 7, 6 , 5 or 4 days of treatment), the dosage of said S1 P receptor modulator or agonist is given at a dosage of up to 10-fold less, e.g. up to 5-fold less, than the standard daily dose, e.g. the therapeutic dose. Optionally the dose is then raised stepwise up to the standard daily dose, e.g. the therapeutic dose, during the initial period of treatment as herein above defined.

Preferred medications comprise medication for patients suffering from chronic long term diseases, such as autoimmune diseases, e.g. multiple sclerosis, Polymyositis, lupus nephritis, rheumatoid arthritis, inflammatory bowel diseases or psoriasis. In an embodiment of the invention, medications are medications for patients suffering from multiple sclerosis, for example relapse remitting multiple sclerosis (RRMS) or primary progressive multiple sclerosis (PPMS), e.g. for patients suffering from RRMS.

The dosage regimen of the present invention is particularly useful for treating patents at risk of cardiac side effects, for example patients at risk of heart failure, arrythmias, patients with high grade atrio-ventricular blocks or sick sinus syndrome, patients with a history of syncopal episodes, or patients requiring or under beta blockers, or patients requiring or under anti-arrhythmic treatment, such as patients under treatment with Class la (e.g. quinidine, procainamide) or Class III anti-arrhythmic drugs (e.g., amiodarone, sotalol).

For compound A (or B), an example of standard daily dosage may be a daily dosage of up to 5mg, for example, the dosage may be between 0.5 and 5 mg, e.g. between 0.5 and 2mg. In a specific embodiment, the standard daily dosage is about 1.25 mg. In another embodiment, the standard daily dosage is about 0.5 mg.

Preferably, the dosage of the S1 P receptor modulator or agonist during the initial period of treatment is increased stepwise in defined increments up to the standard daily dosage of the S1 P receptor modulator or agonist. Preferably, the dosage of said S1 P receptor modulator or agonist during the initial period of treatment as hereinabove defined, e.g. during the initial 10 days, e.g. 1 to 9 days, e.g. first week of treatment is increased in increments of about 1.5- to about 3.5-fold, for example from 2 to 3-fold, for example 2-fold, for example about 1.5-fold.

For example, during the initial period, the dose may be about 10-fold less, or about 8-fold less, or about 5-fold less, or about 3-fold less, about 2-fold less or about 1.5-fold less than the standard daily dosage, e.g. than the therapeutic dose. Suitably the initial dosage administered during the initial period of treatment is about 10-fold less than the standard daily dosage, e.g. than the therapeutic dose, and is then increased in increments to a daily dosage which is about 5 fold less than the standard daily dosage and then about 2.5 fold less than the standard daily dosage. Thereafter, treatment is commenced at the standard daily dosage.

In another embodiment of the invention, the initial dosage administered during the initial period of treatment as hereinabove defined is about 4-fold less than the standard daily dosage, e.g. than the therapeutic dose, and is then increased in increments to a daily dosage which is about 2 fold less than the standard daily dosage. Thereafter, treatment is commenced at the standard daily dosage.

The same dose may be given during the first 1, 2, 3, 4, 5, 6, 7 or 8 days of treatment before the dosage is increased, preferably during the first 2 to 4 days of treatment. Suitably each subsequent incremental dosage increase is administered for 1, 2, 3, 4 or 5 days. In a particular embodiment, each subsequent incremental dosage increase is administered for 2 or 3 days.

During the initial period of treatment, i.e. before the standard daily dosage is given, the same initial dosage may be given during the first 1 to 7 days, e.g. for 2 to 5 days, e.g. the first 2 days, before the dosage is further increased, e.g. up to the standard daily dosage in one or more increments.

One or more dosage increases, e.g. up to 10 dosage increases, e.g. up to 8 dosage increases, e.g. up to 6 dosage increases, e.g. up to 5 dosage increases, up to 4 dosage increases, up to 3 dosage increases, may be performed until the standard daily dosage is given. For example 1 to 10, e.g. 1 to 8, e.g. 1 to 3, e.g. 2 to 8, e.g. 3 to 6 dosage increases may be given.

In an embodiment, the daily dosage is governed by a Fibonacci series i.e. the dosage given on a specific day is the sum of the dosages on the previous two days. In an aspect of this embodiment, some variation in this scheme is permitted. For example, the dosage on a given day may be the sum of the dosages on the two previous days ± 40%, for example ± 30%, for example ±20% or ± 10%.

For example, the first dosage increase may occur on day 2 to day 5, e.g. day 2 to day 4, e.g. day 2, day 3, day 4 or day 5, after the first administration. The second dosage increase, if any, may occur on day 4 to 10, e.g. day 4 to 6, e.g. day 5, after the first administration. The third dosage increase, if any, may occur on day 6 to 10, e.g. day 6 or 7, after first administration.

In one embodiment of the invention, only one or two dosage increase occur before the standard daily dosage, e.g. the therapeutic dosage, is given.

In a particular embodiment, the S1 P receptor agonist is Compound A or Compound B, e.g. FTY720, or a pharmaceutically acceptable salt thereof, e.g. the hydrochloride salt of FTY720.

According to a preferred embodiment of the invention, the highest initial dosage of the S1 P receptor modulator or agonist (e.g. Compound A, Compound B or a salt thereof) is between 0.01 mg and 0.30 mg, e.g. between 0.125 and 0.25mg, preferably 0.125mg or 0.1 mg.

A particularly preferred dosage range of the S1 P receptor modulator or agonist (e.g. Compound A,Compound B or a salt thereof, e.g. the hydrochloride salt of FTY720) during the initial period of treatment as hereinabove defined is e.g. 0.125-1.25 mg, or 0.1-0.5 mg, or 0.125-0.5 mg, or 0.25-0.5 mg.

For example it may be a regimen of 0.125mg/0.25mg/0.5mg, respectively, during the initial period of e.g. up to the first 10 days. Thereafter the treatment is continued with the standard daily dosage, e.g. a dosage of 1.25 mg. This regimen is particularly adapted for compound A.

Alternatively, it may be a regimen comprising an initial daily dose of 0.125mg which is subsequently increased to a daily dose of 0.25mg during the initial period of treatment. Thereafter the treatment is continued with the standard daily dosage, e.g. 0.5mg. Again, this regimen is particularly adapted for compound A.

In a series of further specific or alternative embodiments, the present invention also provides:
1.1 The use of a S1 P receptor modulator or agonist which induces a negative chronotropic effect in heart rate, e.g. compound A, or B, or a salt or prodrug thereof, preferably compound A, a salt or prodrug thereof, in the manufacture of a medication, whereby said medication is administered in such a way to a subject that the daily decrease in heart rate (e.g. the average daily heart rate) is approximatively of 2 beats/min or less.
1.2 The use of a S1 P receptor modulator or agonist, e.g. compound A, or B, or a salt or prodrug thereof, preferably compound A, a salt or prodrug thereof, in the manufacture of a medication, whereby said medication is administered in such a way to a subject that at the day the therapeutic dosage of said S1 P receptor modulator or agonist is administered the decrease in heart rate (e.g. the average daily heart rate) is approximatively of 2 beats/min or less.
1.3 The use of a S1 P receptor modulator or agonist, e.g. compound A, or B, or a salt or prodrug thereof, preferably compound A, a salt or prodrug thereof, in the manufacture of a medication, whereby said medication is administered at a lower dosage than standard dosage, e.g. up to 10-fold less, e.g. 4-fold less, than the standard daily dosage, during the initial period, e.g. during the first 10 days of treatment or during the first week of treatment. Optionally the dosage is then increased stepwise up to the standard daily dosage, e.g. the therapeutic dosage, of said S1 P receptor agonist.
   During the initial period of treatment, e.g. the initial 10 days, e.g. 9 days, e.g. a the first week, of treatment, the daily dosage of the S1 P receptor modulator or agonist is lower than the standard dosage, and is raised stepwise up to 6 times, e.g. three times, e.g. two times,up to the standard daily dosage of said S1 P receptor modulator or agonist and thereafter the treatment is continued with the standard daily dosage of said S1 P receptor modulator or agonist.
1.4. The use of a S1 P receptor modulator or agonist, e.g. compound A, or B, or a salt or prodrug thereof, preferably compound A, a salt or prodrug thereof, in the manufacture of a medication, whereby said medication is administered in such a way that during the initial period of treatment, e.g. the initial 10 days of treatment, e.g. the initial first week of treatment, the dosage of said S1 P receptor modulator or agonist is 10; 5; and 2-3 fold less than the standard daily dosage, respectively, and thereafter the treatment is continued with the standard daily dosage of the S1 P receptor modulator or agonist.
1.5 The use of a S1 P receptor modulator or agonist, e.g. compound A, or B, or a salt or prodrug thereof, preferably compound A, a salt or prodrug thereof, in the manufacture of a medication, whereby said medication is administered in such a way that during the initial period of treatment, e.g. the initial 10 days of treatment, e.g. the initial first week of treatment, the dosage of said S1 P receptor modulator or agonist is 4; and 2 fold less than the standard daily dosage, respectively, and thereafter the treatment is continued with the standard daily dosage of the S1 P receptor modulator or agonist.
1.6 The use of a S1 P receptor modulator or agonist, e.g. compound A, or B, or a salt or prodrug thereof, preferably compound A, a salt or prodrug thereof, in the manufacture of a medication, whereby said medication is administered in such a way that during the initial period of treatment, e.g. the initial 10 days of treatment, e.g. the initial first week of treatment, the dosage of said S1 P receptor modulator or agonist increases from an initial dosage which is 10 fold less than the standard daily dosage to a dosage which is 1.5-3 or 2-3 fold less than the standard daily dosage, and thereafter the treatment is continued with the standard daily dosage of the S1 P receptor modulator or agonist.
1.7. The use of a S1 P receptor modulator or agonist, e.g. compound A, B, or a salt or prodrug thereof, preferably compound A, or a salt or prodrug thereof, in the manufacture of a medication, whereby said medication is administered in such a way that during the initial 2 to 4 days of treatment the dosage of said S1 P receptor modulator or agonist is not more than 1/10, or not more than ¼, of the standard daily dose of the S1 P receptor modulator or agonist.
1.8 The use of a S1 P receptor modulator or agonist, e.g. compound A, B, or a salt or prodrug thereof, preferably compound A, or a salt or prodrug thereof, in the manufacture of a medication, whereby said medication is administered in such a way that during the initial 2 to 4 days of treatment the dosage of said S1 P receptor modulator or agonist is not more than 10% or not more than 25% of the standard daily dose of the S1 P receptor modulator or agonist.
1.9 The use of an S1 P receptor modulator or agonist, e.g. compound A, B, or a salt or prodrug thereof, in the manufacture of a medication, whereby said medication is administered in such a way that during the initial 10 days, e.g. 9 days, of treatment the dosage of said S1 P receptor modulator or agonist is lower than the standard daily dosage of said S1 P receptor modulator or agonist and then the dosage is raised so that the standard daily dosage is administered after several dose increases, up to 10, e.g. up to 6, e.g. two or three dose increases, and thereafter the treatment is continued with the standard daily dosage of said S1 P receptor agonist.
1.10 The use of a S1 P receptor modulator or agonist, e.g. compound A, or B, or a salt or prodrug thereof, preferably compound A, a salt or prodrug thereof, in the manufacture of a medication, whereby said medication is administered in such a way to a subject that the possible risk of AV block is limited or reduced to a level clinically not significant. Preferably the use is then as defined under 1.1 to 1.9.
1.11 The use of a S1 P receptor modulator or agonist, e.g. compound A, or B, or a salt or prodrug thereof, preferably compound A, a salt or prodrug thereof, in the manufacture of a medication, whereby said medication is administered in such a way to a subject that the sinus rhythm of the patient is normal. Preferably the use is then as defined under 1.1 to 1.9.
1.12 Use as defined under 1.1 to 1.11, wherein the initial period of treatment is of up to 10 days, e.g. up to 8 days , e.g. a week.
1.13 The use as defined under 1.1 to 1.12 wherein the medication is given to a patient who is at risk of heart failure.
1.14 The use as defined under 1.1 to 1.12 wherein the medication is given to a patient who is at risk of AV block.
1.15 The use as defined under 1.1 to 1.12 wherein the medication is given to a patient who experience symptoms including dizziness, fatigue, palpitations.
1.16 The use as defined under 1.1 to 1.12 wherein the medication is given to a patient with high grade atrio-ventricular blocks or sick sinus syndrome.
1.17 The use as defined under 1.1 to 1.12 wherein the medication is given to a patient with arrhythmias, e.g. requiring or under treatment with Class la (e.g. quinidine, procainamide) or Class III anti-arrhythmic drugs (e.g. amiodarone, sotalol).
1.18 The use as defined under 1.1 to 1.12 wherein the medication is given to a patient requiring or under beta-blocker therapy.
1.19 The use as defined under 1.1 to 1.18 wherein the medication is given to a patient, e.g. a patient suffering from multiple sclerosis, wherein the administration of said S1 P receptor modulator or agonist, e.g. compound A, or B, or a salt or prodrug thereof, preferably compound A, a salt or prodrug thereof, has been discontinuedformore than 10 days, e.g. more than 12 days, e.g. more than 14 days.
1.20 The use of FTY720, a salt or prodrug thereof in the manufacture of a medication, whereby said medication is administered, after an initial regimen as hereinabove defined, at a daily dosage of about 1.25 mg, or about 0,5mg or less as herein above defined.
1.21 Use as defined in 1.1 to 1.20, for treating a chronic long term diseases, such as an autoimmune disease, e.g. multiple sclerosis, e.g. RRMS.
1.22 Use of FTY720 (Compound A), Compound B, or a pharmaceutically acceptable salt thereof, in the manufacture of a medicament for use in the treatment of autoimmune diseases, wherein, prior to commencing the administration of FTY720, Compound B, or a pharmaceutically acceptable salts thereof, at the standard daily dosage, the FTY720, Compound B, or a pharmaceutically acceptable salts thereof, is administered at a daily dosage which is lower than the standard daily dosage during an initial period of treatment as herein above defined (e.g. up to 10 days).
1.23 The use as defined in 1.22 wherein the standard daily dosage is about 0.5mg to about 1.25mg.
1.24 The use as defined in 1.23 wherein an initial daily dose of 0.125 mg is administered which is subsequently increased to 0.25 mg and then further increased to 0.5 mg during the initial period of treatment, and thereafter treatment is continued at the standard daily dosage, e.g. 1.25 mg.
1.25 The use as defined in 1.23 wherein an initial daily dose of 0.125 mg is administered which is subsequently increased to 0.25 mg during the initial period of treatment and thereafter treatment is continued with the standard daily dosage, e.g. 0.5 mg.

In a treatment method with a S1 P receptor modulator or agonist, e.g. compound A, B or a salt or prodrug thereof, the improvement is due to said S1 P receptor modulator or agonist is administered in such a way that during the initial period of treatment, e.g. the initial 10 days, e.g. 9 days, e.g. first week, of treatment, the dosage is lower than the standard dosage, e.g. 10-fold less, e.g. 5-fold less, e.g. 4 fold-less, e.g. 2 to 3 fold-less, e.g. 2 fold-less, than the standard daily dosage, and is increased, optionally stepwise, up to the standard daily dosage. Thereafter the treatment is continued with the standard effective daily dosage.

Furthermore there is provided:
2.1 A method for treating a patient in need thereof such a method comprising administering a S1 P receptor modulator or agonist which induces a negative chronotropic effect in heart rate, e.g. compound A, B, or a salt or prodrug thereof, comprising administering to the subject a S1 P receptor modulator or agonist in such a way that at the day the therapeutic dosage is administered the decrease in heart rate (e.g. the average daily heart rate) is clinically not significant, preferably is limited to approximatively 2 beats/min or less.
2.2 A method as defined under 2.1 comprising administering to the subject sub-therapeutic doses of the S1 P receptor agonist during the initial period of treatment.
2.3 A method for treating a chronic long term diseases as herein above defined, e.g. an autoimmune disease, e.g. multiple sclerosis, e.g. RRMS, in a subject in need thereof, comprising administering to the subject, a loading regimen of a S1P receptor modulator or agonist, e.g. compound A, B, or a salt or prodrug thereof, at a daily dosage which is lower than the standard daily dosage.
2.4 A method for treating a chronic long term diseases as herein above defined, e.g. an autoimmune disease, e.g. multiple sclerosis, e.g. RRMS, in a subject in need thereof, comprising administering to the subject, a S1 P receptor modulator or agonist, e.g. compound A, B, or a salt or prodrug thereof, at a daily dosage which is lower than the standard daily dosage and raising the daily dosage stepwise up to the standard daily dosage during the initial period of treatment, e.g. the first 10 days.
2.5 A method for treating a chronic long term diseases as herein above defined, e.g. an autoimmune disease, e.g. multiple sclerosis, e.g. RRMS, in a subject in need thereof, comprising administering to the subject, an initial regimen up to 10-fold less the standard daily dosage, and thereafter the daily dosage of a S1 P receptor modulator or agonist, e.g. compound A, B, or a salt or prodrug thereof.
2.6 A method of ameliorating or preventing a negative chronotrophic side effect associated with a treatment using an S1 P modulator or agonist, e.g. compound A, B, or a salt or prodrug thereof, of a subject suffering from an autoimmune disease, comprising administering to the subject in need thereof, said S1 P receptor modulator or agonist at a daily dosage which is lower than the standard daily dosage during an initial treatment period and raising the daily dosage stepwise up to the standard daily dosage.
2.7 A method as defined under 2.1 to 2.6 whereby the initial period of treatment is of up to 10 days, e.g. up to 8 days , e.g. a week.
2.8 A method as defined under 2.1 to 2.6 wherein the initial treatment period is 6-14 days e.g. 7-10 days e.g. 7 days or less, as herein above described.
2.9. A method as defined under 2.1 or 2.8 for treating an autoimmune disease, e.g. multiple sclerosis.
2.10 The method as defined in 2.1 to 2.9, wherein the S1 P receptor modulator or agonist, is FTY720 or a pharmaceutically acceptable salt thereof, e.g. hydrochloride salt, or FTY720 phosphate, and is administered, after an initial regimen, at a daily dosage of about 1.25 mg, or about 0,5mg or less as herein above defined.
2.11 A method of treating a chronic long term diseases as herein above defined, e.g. an autoimmune disease, e.g. multiple sclerosis, e.g. RRMS, in a patient in need of such treatment, the method comprising initiating treatment with the S1 P receptor modulator or agonist at a daily dosage which is lower than the standard daily therapeutic dosage during an initial period of treatment and thereafter commencing the administration of the S1 P receptor modulator or agonist at the required standard daily therapeutic dosage.
2.12 A method of ameliorating or preventing a negative chronotrophic side effect associated with a treatment of an autoimmune disease using an S1 P modulator or agonist, e.g. compound A, B, or a salt or prodrug thereof, the method comprising administering the S1 P receptor modulator or agonist at a daily dosage which is lower than the standard daily dosage during an initial treatment period and raising the daily dosage, optionally stepwise, up to the standard daily dosage.
2.13 A method of treating a chronic long term diseases as herein above defined, e.g. an autoimmune disease, e.g. multiple sclerosis, e.g. RRMS, in a patient who is at risk of heart failure, the method comprising administering the S1 P receptor modulator or agonist, e.g. compound A, B, or a salt or prodrug thereof.
2.14 A method of treating a chronic long term diseases as herein above defined, e.g. an autoimmune disease, e.g. multiple sclerosis, e.g. RRMS, in a patient who is at risk of AV block, with high grade atrio-ventricular blocks or sick sinus syndrome, the method comprising administering the S1 P receptor modulator or agonist, e.g. compound A, B, or a salt or prodrug thereof.
2.15 A method of treating a chronic long term diseases as herein above defined, e.g. an autoimmune disease, e.g. multiple sclerosis, e.g. RRMS, in a patient who experiences symptoms including dizziness, fatigue, palpitations, the method comprising administering the S1 P receptor modulator or agonist, e.g. compound A, B, or a salt or prodrug thereof.
2.16 A method of treating a chronic long term diseases as herein above defined, e.g. an autoimmune disease, e.g. multiple sclerosis, e.g. RRMS, in a patient with arrhythmias, e.g. requiring or under treatment with Class la (e.g. quinidine, procainamide) or Class III anti-arrhythmic drugs (e.g. amiodarone, sotalol); or in a patient requiring or under beta-blocker therapy, the method comprising administering the S1 P receptor modulator or agonist, e.g. compound A, B, or a salt or prodrug thereof.
2.17 A method of treating a chronic long term diseases as herein above defined, e.g. an autoimmune disease, e.g. multiple sclerosis, e.g. RRMS, while limiting the occurrence of symptoms including dizziness, fatigue, heart palpitations, the method comprising administering the S1 P receptor modulator or agonist, e.g. compound A, B, or a salt or prodrug thereof.
2.18 A method as defined in 2.11 or 2.17 wherein the S1 P receptor modulator or agonist is selected from Compound A (FTY720), or a pharmaceutically acceptable salt thereof, and FTY720 Phosphate (Compound B), or pharmaceutically acceptable salts thereof.

In yet another embodiment of the invention, there is provided
3.0 A S1 P receptor modulator or agonist for use in the treatment of a chronic long term diseases as herein above defined, e.g. an autoimmune disease, e.g. multiple sclerosis, e.g. RRMS, wherein, prior to commencing the administration of the S1P receptor modulator or agonist at the standard daily therapeutic dosage, said S1 P receptor modulator or agonist is administered at a daily dosage which is lower than the standard daily therapeutic dosage during an initial period of treatment.
4.0 A kit containing daily units of medication of an S1 P receptor modulator or agonist, e.g. compound A, B, or a salt or prodrug thereof, of varying daily dosage, whereby said doses are lower than the standard daily dosage.
4.1 A kit comprising units of medication of a S1 P receptor modulator or agonist as defined herein for administration according to the dosage regimen defined herein, whereby one or more low-dose units of a dose strength below the standard daily dose of the S1 P receptor agonist are provided for the initial period of treatment as herein above defined, e.g. the frst week of treatment.
4.2 A kit containing units of medication of an S1 P receptor modulator or agonist as defined herein of varying daily dosage, whereby said kit contains a) at least one of the following : about 1/10, about 1/8, about 1/5, about %, about 1/3, about ½.5, about ½, about 1/1.5, of the standard dose of the S1 P receptor modulator or agonist, respectively, and b) optionally units for the standard daily dosage of the S1 P receptor modulator or agonist.

In an embodiment, the kit may comprise just one low dose unit of medication at a dosage strength corresponding to an initial dosage of the S1 P receptor modulator or agonist. A patient may then take one unit of the low dose medication for a specified number of days and then, optionally, two or more units per day on subsequent days until therapy is commenced with a unit of medication that comprises the standard daily dose of the S1 P receptor agonist.

In an alternative embodiment, the kit may comprise a number of low-dose units of medication with a range of dosage strengths so that the patient can be administered one dosage unit per day, but the amount of S1 P receptor modulator or agonist administered can be titrated upwards until therapy commences at the standard daily dosage.

For example, the daily units of said S1 P receptor modulator or agonist may be of about 1/10, about 1/5 and about 1/2.5; or about 1/5 and about 1/2.5, of the standard dose of said S1 P receptor modulator or agonist, respectively.

In another embodiment, the daily units of said S1 P receptor modulator or agonist may be of about 1/8, about 1/4 and about ½; or about 1/4 and about ½, of the standard dose of said S1 P receptor modulator or agonist, respectively.

The kit may further comprise units for the standard daily dosage of the S1 P receptor modulator or agonist, e.g. compound A, B, or a salt or prodrug thereof.

The kit may also contain instructions for use.

In yet another embodiment of the invention, there is provided
5.1 A method for treating a chronic long term diseases as herein above defined, e.g. an autoimmune disease, e.g. multiple sclerosis, e.g. RRMS, , or a method for treating an autoimmune disease in a subject in need thereof in a subject in need thereof, comprising administering to the subject, a daily dosage of FTY720 or a pharmaceutically acceptable salt thereof, e.g. of about 1.25 mg, or about 0,5mg or less as herein above defined.
5.2. The method as defined in 5.1, wherein the disease is multiple sclerosis, e.g. RRMS.
5.3. The method as defined in 5.1, wherein the autoimmune disease is multiple sclerosis.
6.1 A method for assessing the need or suitability of a patient for a treatment regimen as described above (e.g. in any of the specified aspects or embodiments of the invention), comprising the steps of:
   (i) determining whether the patient to be treated with an S1P receptor modulator or agonist is in a category for which the use of a treatment regimen as described above may be beneficial; and
   (ii) if the patient falls within this category, treating the patient using a treatment regimen as described above.
6.2 The method as defined in 6.1 wherein the patient may be in the above category if he or she suffers from or is susceptible to heart failure, arrhythmias, high grade atrio-ventricular blocks or sick sinus syndrome or has a history of syncopal episodes; or is undergoing beta blocker or anti-arrhythmic treatment, e.g. is under treatment with anti-arrhythmic drugs; or has undergone an interruption or treatment holiday in the maintenance dosage regime e.g. a holiday of greater than 4 days, greater than 6, 8, 10, 12 or 14 days.

The regimen of S1 P receptor modulator or agonist which is administered to the subject according to the invention may be given either during at the beginning of the disease therapy, e.g. during the initial 10 days, or after an interruption of S1 P receptor modulator or agonist therapy, for example an interruption of more than 10 days, more than 12 days, e.g. more than 14 days.

In a further aspect, the present invention relates to a daily dosage of the S1 P receptor modulator or agonist, e.g. compound A (or B), or, in each case, a pharmaceutically acceptable salt, e.g. a hydrochloride salt, thereof, is selected from any one of the following: about 1.25 mg or less, e.g. is from about 1.25 mg to about. 0.01 mg, e.g. is 1.25 mg, e.g. 1.20 mg, e.g. 1.15 mg, e.g. 1.10 mg, e.g. 1.05mg, e.g. 1.00 mg, e.g. 0.95mg, e.g. 0.90 mg, e.g. 0.85mg, e.g. 0.80 mg, e.g. 0.75 mg, e.g. 0.70 mg, e.g. 0.65 mg, e.g. 0.60 mg, e.g. 0.55 mg, e.g. 0.50 mg, e.g. 0.45 mg, e.g. 0.40 mg, e.g. 0.35 mg, e.g. 0.30 mg, e.g. 0.25mg, e.g. 0.20 mg, e.g. 0.15 mg, e.g. 0.125 mg, e.g. 0.12 mg, e.g. 0.115 mg, e.g. 0.11mg, e.g. 105 mg, e.g. 0.1 mg, e.g. 0.055 mg, e.g. 0.05 mg, e.g. 0.045 mg, e.g. 0.04 mg, e.g. 0.035 mg, e.g. 0.03 mg, e.g. 0.025 mg, e.g. 0.02 mg, e.g. 0.01 mg.

Preferably the daily dosage of the compound, e.g. FTY720 or FTY720 phosphate, is 0.5mg.

The present invention therefore includes, in a further aspect, the above mentioned daily dosages of FTY720, FTY720 phosphate or, in each case, a pharmaceutically acceptable salt, e.g. a hydrochloride salt, thereof. In particular, the invention relates to the above mentioned daily dosages of FTY720 phosphate or the hydrochloride salt of FTY720.

In a specific embodiment the daily dosage of FTY720, FTY720 phosphate or, in each case, a pharmaceutically acceptable salt, e.g. a hydrochloride salt, is about 0.5 mg, or about 0.25mg, or about 0.125 mg, or about 0.1 mg.. In another embodiment the daily dosage of FTY720 phosphate or the hydrochloride salt of FTY720 is about 0.5 mg, or about 0.25mg, or about 0.125 mg, or about 0.1 mg.

The utility of an S1 P receptor modulator or agonist dosage regimen in treating diseases and conditions as hereinabove specified may be demonstrated in standard animal or clinical tests, e.g. in accordance with the methods described hereinafter.

### Brief description of the Figure:

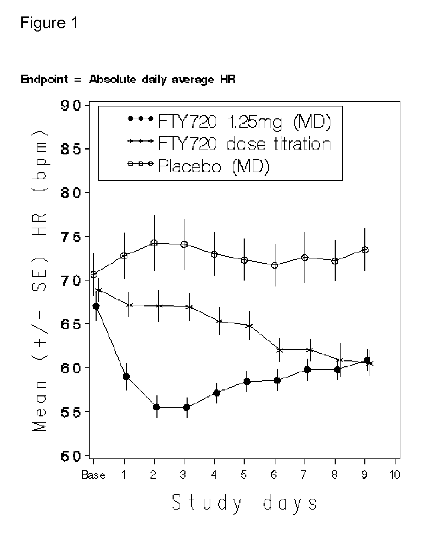

Figure 1 shows the mean (+/- standard error) daily average heart rate on days 1 to 9 for the treatment regimen groups described in the Example below. The x-axis is shows the study days and the Y-axis shows the mean (+/-SE) daily average heart rate (beats per minute (BPM)).

### Example:

A single-center, double-blind, placebo-controlled, dose titration, once-daily, multiple-oral-dose clinical study in healthy subjects was conducted to evaluate the effect of a dosage regimen of FTY720 according to the present invention.

A total of 36 subjects were randomly assigned to one of the three groups.

Each subject participated in a screening period of maximal 21 days, a baseline period (Day-1), a 9-day dose-administration period, and clinical study completion assessments on Day 10. Subjects were assigned to one of the three groups (dose titration regimen group, placebo control group, or active control group) and received the once daily dose in a double-blinded manner (Table 1).

In the dose titration group, increasing once daily doses of Compound A (FTY720), starting at 0.125 mg o.d. and ending at the maximal therapeutic dose of 1.25 mg o.d. were administered on days 1 to 9 of the study according to the schedule shown in Table 1 below. The placebo control group received placebo through out the duration of the study and the active control group received a once daily dose of 1.25 mg of FTY720 on days 1 to 9.

**Table 1**

| Study group / Number of subjects | Baseline | Administration period | | | | | | | | |
|---|---|---|---|---|---|---|---|---|---|---|
| Day | Day -1 | Day 1 | Day 2 | Day 3 | Day 4 | Day 5 | Day 6 | Day 7 | Day 8 | Day 9 |
| Dose titration regimen group / N = 15 | Placebo | 0.125 mg | 0.125 mg | 0.125 mg | 0.25 mg | 0.25 mg | 0.5mg | 0.5mg | 1.25 mg | 1.25 mg |
| Placebo control group / N=9 | Placebo | Placebo, once daily over 9 days | | | | | | | | |
| Active control group / N = 12 | Placebo | 1.25 mg, once daily over 9 days | | | | | | | | |

On Day -1 (baseline), subjects will undergo baseline assessments including 24 hour holter monitoring and telemetry assessments.

Pharmacodynamic and safety assessments are performed up to 24 hours post last dose. Heart rate is monitored via telemetry on Day -1 through Day 10, 24 hour after the last dosing. Heart rhythm is assessed via 24hr continuous holter monitoring on Day -1, Day 1, and Day 9. For each dosing for each subject, the drug is administered as closely as practically possible to the time administered on Day -1. Safety assessments includes physical examinations, vital signs and body measurements, 12-lead ECG evaluations, standard clinical laboratory evaluations hematology, blood chemistry, urinalysis, adverse event and serious adverse event monitoring. Systolic and diastolic blood pressure and pulse are measured after the subject has rested quietly in the sitting position for at least 3 minutes. Blood pressure is measured at the same arm on each occasion. Standard 12-lead ECGs are recorded at Screening, baseline, 4 hours post-dose on Day 1 and Day 8, and at Study Completion. The variables recorded are: date and time of ECG, heart rate, PR interval, QT interval (uncorrected), QTcB, QRS duration. The overall interpretation is collected with a Yes/No statement to confirm if any clinically relevant abnormalities are present, which needs to be specified further.

Subjects remain on continuous telemetry, starting before breakfast on Day -1 and proceeding throughout the administration period up to Day 10 (24 hour after the last dose). Over this ten day duration of continuous heart rate collection for each subject, one heart rate value is obtained every minute ('minute unit heart rate'), representing the average heart rate value over that minute. The heart rate database for each subject contains approximately 14,400 data points (10 days x 24 hr x 60 min).

24-hour continuous Holter-ECG data are captured via a digital Holter monitor (12-lead, on Days -1, 1, 6, and 8), and transferred for interpretation and reporting. Holter monitoring starts approximately at 7:00 and the time of dose administration is regarded as the time "0 hours". Holter "cuts" are derived from the dataset at 1 hour intervals starting from Day -1 and continuing for 24 hours or the end of the cleaned Holter monitoring dataset.

Cardiac conduction intervals: arrhythmia monitoring includes the frequency and duration of sinus pauses (>2 sec and > 3 sec) and atrio-ventricular blocks. Frequency and duration of atrial and ventricular ectopy and sinus rhythm are also recorded. The daily chronotropic effect is defined as the percent decrease in HRₘᵢₙ (minimum heart rate) between two consecutive days. It is calculated on days 1 to 9.

### Results:

The results are shown in Figure 1. In the placebo group, daily average heart rate varies by approximately 5 BPM (beats per min) over the course of the study with a trend for heart rate to increase approximately 3-4 BPM from Day -1 to Day 2. The FTY720 1.25 mg treatment group manifestes a significant decrease in heart rate of approximately 8 BPM from Day -1 to Day 1 and an additional decrease in heart rate approximately 3 BPM from Day 1 to Day 2. The FTY720 titration group manifestes a gradual decrease in heart rate of approximately 1-2 BPM per day over the course of eight day course of the dose titration. The initiation of the 1.25 mg FTY720 on Day 8 does not result in dip in heart rate compared to the preceding days.

These results indicate that the use of a dose titration regimen according to the invention attenuates the negative chronotropic effect seen on Day 1 of FTY720 treatment initiation. Furthermore multiple analyses have been conducted to compare minimum heart rates between the two FTY720 treatment group. These analysis show that the FTY720 dose titration regimen provides improved daily minimum heart rates during the course of the study.

## Claims

1. Use of a S1 P receptor modulator or agonist in the manufacture of a medicament for use in the treatment of a long chronic disease in a patient whereby said medicament is administered in such a way to a subject that the daily decrease in heart rate is approximatively of 2 beats/min or less.

2. Use of a S1 P receptor modulator or agonist in the manufacture of a medicament for use in the treatment of a long chronic disease , wherein, prior to commencing the administration of the S1 P receptor modulator or agonist at the standard daily therapeutic dosage, said S1 P receptor modulator or agonist is administered at a daily dosage which is lower than the standard daily therapeutic dosage during an initial period of treatment.

3. The use according to claim 1 or 2, comprising increasing, optionally stepwise, the administered dosage during the initial period of treatment up to the standard daily dosage of said S1 P receptor modulator or agonist.

4. The use according to any preceding claim wherein the daily dosage during the initial period of treatment is up to 10 fold less than the standard daily dosage.

5. The use according to any preceding claim, wherein the initial period of treatment is selected from the group consisting of: 4 to 12 days, 5 to 14 days, up to 10 days, 7 to 10 days, 9 days, 8 days, 7 days, 6 days, 5 days or 4 days.

6. Use of a S1 P receptor modulator or agonist in the manufacture of a medicament for use in the treatment of a long chronic disease, e.g. according to claim 1 to 5, in a patient at risk of cardiac side effects or heart failure.

7. Use of a S1 P receptor modulator or agonist in the manufacture of a medicament for use in the treatment of a long chronic disease, e.g. according to claim 1 to 5, while limiting, reducing of preventing the occurrence of symptoms including dizziness, fatigue and heart palpitations,

8. A method of treating a long chronic disease in a patient in need of such treatment, the method comprising initiating treatment with the S1 P receptor modulator or agonist at a daily dosage which is lower than the standard daily therapeutic dosage during an initial period of treatment and thereafter commencing the administration of the S1 P receptor modulator or agonist at the required standard daily therapeutic dosage.

9. A method of ameliorating, preventing or limiting a negative chronotrophic side effect associated with a treatment of an autoimmune disease using an S1 P modulator or agonist, e.g. compound A, B, or a salt or prodrug thereof, the method comprising administering the S1 P receptor modulator or agonist at a daily dosage which is lower than the standard daily dosage during an initial treatment period and raising the daily dosage, optionally stepwise, up to the standard daily dosage.

10. Use or method according to any preceding claim wherein the S1P receptor modulator or agonist is selected from Compound A (FTY720), or a pharmaceutically acceptable salt thereof, and Compound B (FTY720 Phosphate), or pharmaceutically acceptable salts thereof.

11. A kit comprising units of medication of a S1 P1 receptor modulator or agonist for administration according to the dosage regimen defined in any one of claims 1 to 7 wherein one or more low-dose units of a dose strength below the standard daily dose of the S1 P receptor agonist are provided for the initial period of treatment.

12. A kit containing daily units of medication of an S1 P receptor modulator or agonist of varying daily dosage, whereby the daily dosage of S1 P receptor modulator or agonist is about 1/5 and about 1/2.5; or about % and about ½, of the standard dose of the S1P receptor modulator or agonist, respectively, and optionally units for the standard daily dosage of the S1 P receptor modulator or agonist.

13. A kit containing daily units of medication of an S1P receptor modulator or agonist of varying daily dosage, whereby said kit contains a) at least one of the following : about 1/10, about 1/8, about 1/5, about %, about 1/3, about ½.5, about ½, about 1/1.5, of the standard dose of the S1 P receptor modulator or agonist, respectively, and b) optionally units for the standard daily dosage of the S1 P receptor modulator or agonist .

14. Use, method of kit according to any preceding claim wherein the disease is multiple sclerosis.

15. Use, method of kit according to any preceding claim wherein the S1 P receptor modulator or agonist is selected from Compound A, a salt thereof, and Compound B.
